# EUROPEAN PATENT SPECIFICATION

(11) **EP 2 046 718 B1**
(45) Date of publication and mention of the grant of the patent: **21.04.2010**
(21) Application number: 07810146.6
(22) Date of filing: 02.07.2007
(51) Int. Cl.: C07C 67/04, C07C 69/013

(54) **PROCESS FOR MAKING TRICYCLODECENYL ESTERS**
VERFAHREN ZUR HERSTELLUNG VON TRICYCLODECENYLESTERN
PROCÉDÉ DE FABRICATION D'ESTERS DE TRICYCLODÉCÉNYLE

(30) Priority: 18.07.2006 US 489100
(43) Date of publication of application: 15.04.2009
(73) Proprietor: Millennium Specialty Chemicals, Inc., Houston, TX 77010 (US)
(72) Inventor: LEBEDEV, Mikhail Y., Jacksonville, Florida 32224 (US)
(74) Representative: De Hoop, Eric
(86) International application number: PCT/US2007/015371
(87) International publication number: WO 2008/010910

(56) References cited:
- US-A- 2 395 452
- US-A- 2 814 639
- US-A- 4 358 617
- WANG, B. ET AL.: "Sulfamic acid as green, efficient, recyclable and reusable catalyst for direct addition of aliphatic acid with cyclic olefins" CATALYSIS LETTERS, vol. 96, no. 1-2, 2004, pages 71-74, XP002462263 cited in the application
- MAMEDOV, M.K.: "Synthesis and reactions of tricyclic monocarboxylic acid esters" RUSSIAN JOURNAL OF ORGANIC CHEMISTRY, vol. 31, no. 4, 1995, pages 485-488, XP009093597 cited in the application
- BRUSON, H.A. ET AL.: "The chemistry of dicyclopentadiene. II. Addition-rearrangement with acids" J. AM. CHEM. SOC., vol. 67, 1945, pages 1178-1180, XP002462264
- HEIDEKUM, A. ET AL.: "Addition of Carboxylic Acids to Cyclic Olefins Catalyzed by Strong Acidic Ion-Exchange Resins" JOURNAL OF CATALYSIS, vol. 181, no. 2, 1999, pages 217-222, XP004443248 ISSN: 0021-9517

## Description

### FIELD OF THE INVENTION

The invention relates to a process for making tricyclodecenyl esters. The esters derive from dicyclopentadiene compounds and are valuable fragrance components.

### BACKGROUND OF THE INVENTION

Tricyclodecenyl esters, particularly the C₂-C₄ esters derived from dicyclopentadiene, are ubiquitous perfume ingredients found in detergents, shampoos, deodorants, hard-surface cleaners, and other applications. The most common tricyclodecenyl ester, which is made by reacting dicyclopentadiene with acetic acid, is tricyclodecenyl acetate or "TCDA." The product, which has a sweet, anise-like aroma, is marketed by several companies, including, for example, International Flavors & Fragrances (Cyclacet®), Quest (Jasmacyclene®), Millennium Specialty Chemicals (Navacet^{™}) and Symrise (Herbaflorat®).

Tricyclodecenyl esters are normally produced by acid-catalyzed addition of the corresponding carboxylic acids (acetic, propionic, butyric, isobutyric) to dicyclopentadiene (DCPD). A large excess of the carboxylic acid is normally used. Solvents, such as aromatic hydrocarbons, are often included. Many catalysts have been proposed, including perchloric acid (U.S. Pat. No. 2,814,639), sulfuric acid (U.S. Pat. No. 2,395,452), boron trifluoride and its addition complexes (U.S. Pat. Nos. 4,855,488 and 4,358,617), sulfamic acid (Catal. Left. 96 (2004) 71), p-toluenesulfonic acid (Zh. Org. Khim. 31 (1995) 528 and Neftek. 37 (1997) 76), and perfluorinated acidic ion-exchange resins (DE 3,105,399).

U.S. Pat. No. 2,395,452 teaches to prepare TCDA using a 550% molar excess of acetic acid and dilute H₂SO₄ as the catalyst. More recently, Leitmannova et al. (Perf. Flav. 29 (2004) 20) optimized the sulfuric acid-catalyzed process and concluded that use of a 400% excess of acetic acid at 100°C provides a favorable balance of product yield and reaction time. Unfortunately, however, acetic acid is relatively expensive and neutralization of the excess acid during workup generates a large amount of waste.

U.S. Pat. No. 4,855,488 teaches to prepare tricyclodecenyl esters from 93%-pure DCPD, a boron trifluoride catalyst, and at least a 200% molar excess of carboxylic acid. Instead of neutralizing the acid, the patentees teach to recover it using a costly stripping step. U.S. Pat. No. 4,358,617 (Example II) starts with a methyl-substituted dicyclopentadiene, BF₃, and only a 25% molar excess of acetic acid, but it includes a large amount of toluene in the reaction mixture and workup.

DE 3,619,797 teaches a process for obtaining TCDA using 1.1 to 5 molar excess of acetic acid and an ion-exchange resin. The water content is 0.5-15% based on the reactor charge, and acetic anhydride is added before distillative workup. In addition to the well-known drawbacks of ion-exchange resins (e.g., cost, loss of activity upon recycle), the need to charge acetic anhydride further complicates the method.

Commercial processes for making tricyclodecenyl esters typically require relatively pure dicyclopentadiene. The commercial grade DCPD material normally used is 93-94% DCPD, although a lower grade (83-88% pure) is available. High-purity DCPD (>98%) is also used. Unfortunately, esters made from the lower grade DCPD may not meet acceptable odor standards or isomer ratio requirements.

Conventional wisdom indicates that carboxylic acids (e.g., acetic acid) used to make tricyclodecenyl esters must also meet minimum purity standards. Crude acetic acid can be recovered from esterification processes used to make fragrance components, but this material is usually contaminated with 20 wt.% or more of acetic anhydride, α-pinene, limonene, acetate esters, and other impurities. Because distillation of such streams is costly, they are often simply discarded.

In sum, a simple, economical way to make tricyclodecenyl esters is needed. A valuable process would overcome cost and waste disposal issues by eliminating the need to use a large excess of the carboxylic acid. Preferably, the method would avoid the additional tradeoffs of using solvents or ion-exchange resins. Ideally, the process would afford high yields of fragrance-quality tricyclodecenyl esters, even if the dicyclopentadiene and/or carboxylic acid sources are relatively impure.

### SUMMARY OF THE INVENTION

The invention is a process for making tricyclodecenyl esters. The process comprises reacting approximately equimolar amounts (0.8 to 1.3 molar ratio) of a C₂-C₄ carboxylic acid and a dicyclopentadiene in the presence of trifluoromethanesulfonic acid (hereinafter "triflic acid") under conditions effective to produce the tricyclodecenyl ester. Surprisingly, the process gives tricyclodecenyl esters in good yield from dicyclopentadienes while avoiding the need to use a large excess of the carboxylic acid. Moreover, commercially acceptable TCDA can be made from an inexpensive grade of DCPD and/or recycled acetic acid. The invention provides an easy, practical, and economical route to fragrance-quality tricyclodecenyl esters.

### DETAILED DESCRIPTION OF THE INVENTION

In the inventive process, a C₂-C₄ carboxylic acid reacts with a dicyclopentadiene. Suitable carboxylic acids are aliphatic carboxylic acids having up to four carbons. Examples include acetic acid, propionic acid, butyric acid, and isobutyric acid. Higher carboxylic acids (C₅ and up) are excluded because the esters do not have much of an odor, while formic acid (C₁) gives esters with a less-desirable odor. Because it is readily available, acetic acid is most preferred.

While any desired grade of acetic acid can be used to make tricyclodecenyl acetates, I surprisingly found that crude acetic acid (80-95 wt.%, with a balance of organic compounds) can provide a fragrance-quality product. Acetic acid and acetic anhydride are staple reagents for the manufacture of esters used as fragrance components. Often, it is impractical to recover the acetic acid by conventional techniques such as extraction or distillation. Consequently, process streams containing acetic acid, acetic anhydride, and organic contaminants are commonplace in the fragrance industry. One such conveniently available stream contains about 80% acetic acid, 10-15% acetic anhydride, α-pinene, limonene, acetate esters, and other impurities. I found that this material can be used instead of glacial acetic acid (99% pure) in the inventive process to give a commercially acceptable tricyclodecenyl acetate (see Example 3, below).

Suitable dicyclopentadienes have a carbon framework derived from dicyclopentadiene. The framework can be substituted with alkyl, halogen, aryl, or other groups that do not interfere with addition of the carboxylic acid to a carbon-carbon double bond of the dicyclopentadiene during preparation of the tricyclodecenyl ester. Preferred dicyclopentadienes are unsubstituted dicyclopentadiene (DCPD) and alkyl-substituted dicyclopentadienes such as the ones disclosed in U.S. Pat. Nos. 4,453,000 and 4,358,617. DCPD is readily available and is most preferred.

The grade of the dicyclopentadiene used is not critical. In fact, this is an advantage of the inventive process because commercial grade DCPD (93-94% pure DCPD) or high-purity DCPD (>98%) is normally needed to achieve desirable fragrance character. Here, however, technical grade DCPD (e.g., >80% pure DCPD such as Lyondell Chemical Company's DCPD-101) can be used as a low-cost alternative with good results. Preferably, the dicyclopentadiene used has a purity within the range of 80 to 90%.

The carboxylic acid and the dicyclopentadiene are combined in roughly equimolar amounts. In particular, up to a thirty mole percent excess of the carboxylic acid can be used, or as little as eighty percent of a molar equivalent. Thus, the molar ratio of carboxylic acid to the dicyclopentadiene is within the range of 0.8 to 1.3. A preferred ratio is from 0.9 to 1.1; more preferred is a ratio within the range of 0.95 to 1.05. The ability to use equimolar amounts of the carboxylic acid and the dicyclopentadiene is another advantage of the invention. Using little or no excess carboxylic acid at the outset can reduce or eliminate the need for subsequent removal of the unreacted portion either by distillation or by neutralization and disposal of the resulting carboxylic acid salt. In contrast, prior-art methods typically require a large excess of the carboxylic acid.

Triflic acid catalyzes the addition reaction between the carboxylic acid and the dicyclopentadiene. The source of the triflic acid is not critical. It can be purchased, for example, from Aldrich Chemical and other suppliers. Only a catalytic amount of triflic acid is needed. Typically, the amount used will be less than 1 wt.% based on the amount of the dicyclopentadiene used. Preferably, less than 0.5 wt.% of triflic acid is used, more preferably less than 0.2 wt.%.

The process is performed under conditions effective to produce tricyclodecenyl esters. By "tricyclodecenyl esters" we mean addition reaction products of one molar equivalent of a C₂-C₄ carboxylic acid and a dicyclopentadiene. The acid adds across one of the two carbon-carbon double bonds of the dicyclopentadiene. Each of the esters has a tricyclic ten-carbon framework that may have additional alkyl or other substituents that derive from the dicyclopentadiene. The tricyclodecenyl esters are normally generated as a mixture of two or more isomers, with one isomer often predominating. Preferably, the tricyclodecenyl ester derives from dicyclopentadiene. Thus, preferred tricyclodecenyl esters include, for Example, tricyclodecenyl acetate (TCDA), tricyclodecenyl propionate, tricyclodecenyl butyrate, and tricyclodecenyl isobutyrate.

Most preferably, the tricyclodecenyl ester is TCDA. Following the reaction of acetic acid and DCPD to produce TCDA, distillation is advantageously used to recover a TCDA-rich product. Commercially acceptable product comprises at least 95 wt.%, more preferably at least 98 wt.%, of TCDA isomers as measured by gas chromatography. The major isomer is preferably at least 90 wt.%, more preferably at least 92 wt.%, of the product. Preferably, the product also has a refractive index (n_{D}²⁰) within the range of 1.49 to 1.50, more preferably from 1.493 to 1.497. Preferred product has a specific gravity (d₄²⁰) within the range of 1.07 to 1.08, more preferably from 1.071 to 1.076.

To be commercially acceptable, the tricyclodecenyl esters usually must also satisfy well-recognized odor requirements. Relatively minor variations in isomer content may or may not impact commercial suitability. Moreover, a particular ester sample may have an acceptable isomers content but an unacceptable odor.

The tricyclodecenyl esters are easy to prepare. The dicyclopentadiene, triflic acid, and acetic acid are combined in any desired order. In one aspect, the dicyclopentadiene is added gradually to a well-agitated mixture of the acetic acid and triflic acid. See Examples 1 and 4.

While the reaction temperature is not critical, it is preferred to perform the reaction at a temperature within the range of 60°C to 150°C, more preferably from 110°C to 140°C. The reaction is normally complete within 1 to 10 hours, depending upon the conditions selected.

The tricyclodecenyl ester is isolated from the reaction mixture by any convenient means. Distillation is particularly preferred. Commercially acceptable esters are normally obtained by distilling the mixture at reduced pressure, preferably < 10 torr, more preferably < 2 torr, and collecting a center cut containing material having an acceptable boiling range.

The process of the invention can be practiced in any desired mode. Thus, a batch, semi-continuous, or continuous process can be employed.

The following examples illustrate the invention.

### EXAMPLE 1

### Preparation of Tricyclodecenyl Acetate using Triflic Acid as a Catalyst

Dicyclopentadiene (DCPD, 1048 g, purity 86%) is added dropwise at 90-110°C to a stirred mixture of glacial acetic acid (420 g) and triflic acid (1.18 g) over 5.7 h. The reaction mixture is then stirred for 2.5 h at 120°C. Brine (98 g, d₄²⁰ 1.202), water (49 g), and 50% aq. NaOH solution (61 g) are added at 50°C, and the mixture stirs for 0.5 h. After the layers settle, the organic phase is isolated and washed with brine (2 x 125 g). Distillation of crude material (1409 g) at 1 torr provides commercial grade TCDA (888 g, 67% based on DCPD). Major isomer by gas chromatography (GC): 92.4%; Isomer A: 1.6%; Isomer B: 4.3%; Isomer C: 0.5%.
Targeted ranges: Major isomer: >90%; Isomer A: <2%; Isomer B: 1-6%; Isomer C: <3%.

### COMPARATIVE EXAMPLE 2

### Preparation of Tricyclodecenyl Acetate using a Boron Trifluoride Catalyst

Dicyclopentadiene (DCPD, 1048 g, purity: 86%) is added dropwise at 90-140°C to a stirred mixture of glacial acetic acid (420 g) and boron trifluoride:acetic acid, 1:2 adduct (15.9 g) over 3.25 h. The reaction mixture is then stirred for 0.75 h at 140°C. Brine (100 g), water (50 g), and 50% aq. NaOH solution (68 g) are added at 50°C, and the mixture stirs for 0.5 h. After the layers settle, the organic phase is isolated and washed with brine (2 x 125 g). Distillation of crude material (1415 g) at 1 torr provides TCDA (786 g, 60% based on DCPD) that is dissimilar to commercial TCDA in terms of isomer content. Major isomer: 90.5%; Isomer A: 4.1%; Isomer B: 3.7%; Isomer C: 1.4%.

Example 1 shows that the inventive process provides a TCDA product that falls within the targeted isomer requirements. When the procedure is modified (Comparative Example 2), too little of the major isomer is made, and too much of Isomer A is produced. An additional comparative experiment demonstrated that commercially acceptable TCDA can be made with the BF₃ catalyst by using a large excess of acetic acid and DCPD having a purity of 93%.

### EXAMPLE 3

### Use of Crude, Recycled Acetic Acid

*Step A. Hydrolysis of acetic anhydride*. A by-product stream containing about 80 wt.% of acetic acid, acetic anhydride (about 13 wt.%), α-pinene, limonene, acetate esters, and other impurities, is combined with enough water (12.3 mL) to hydrolyze the acetic anhydride. Triflic acid (1.36 g) is added, and the mixture is heated with stirring to 110°C and kept at this temperature for 30 min. GC of the resulting dark brown mixture indicates the absence of acetic anhydride, about 90 wt.% of acetic acid, and the balance of a complex mixture of organic compounds.

*Step B. Synthesis of TCDA*. DCPD (1048 g, 86% pure) is added dropwise at 110-120°C to the mixture obtained in Step A over 5.7 hrs. The reaction mixture is stirred for an additional 2.5 h at 120°C and is then cooled to 40°C. Brine (98 g, d₄²⁰ 1.20), water (49 g), and 50% aqueous NaOH (61 g) are added at 50°C and the mixture stirs for 30 min. The layers are separated and the organic phase is washed with brine (2 x 125 g). Crude material (1423 g) is distilled at 1 torr to provide commercial grade TCDA (925 g, 69% based on DCPD).

Example 3 demonstrates that fragrance-quality TCDA can be made from crude, recycled acetic acid and technical grade DCPD.

### EXAMPLE 4

### No Gradual Addition of DCPD

A mixture of DCPD (1048 g, 87-88%), triflic acid (1.19 g), and glacial acetic acid (420 g) is heated with stirring to 130°C over 55 min. The mixture is kept at 130-135°C for an additional 2.4 h, then cooled to 40°C. Brine (104 g, d₄²⁰ 1.20), water (52 g), and 50% aqueous NaOH (66 g) are added at 50°C, and the mixture stirs for 30 min. The phases are separated, and the organic layer is washed with brine (2 x 125 g). Crude material (1284 g) is distilled at 1 torr to provide commercial grade TCDA (690 g, 51% based on DCPD).

Examples 1 and 4 show that the yield of commercially acceptable TCDA is enhanced by gradual addition of DCPD to a mixture of acetic acid and triflic acid.

## Claims

1. A process comprising reacting a C₂-C₄ carboxylic acid and a dicyclopentadiene at a carboxylic acid:dicyclopentadiene molar ratio within the range of 0.8 to 1.3 in the presence of triflic acid under conditions effective to produce a tricyclodecenyl ester.

2. The process of claim 1 wherein the dicyclopentadiene is unsubstituted dicyclopentadiene (DCPD).

3. The process of claim 1 wherein the dicyclopentadiene has a purity within the range of 80 to 90%.

4. The process of claim 1 wherein the tricyclodecenyl ester is distilled to produce a commercially acceptable fragrance component.

5. The process of claim 1 wherein the carboxylic acid is selected from the group consisting of acetic, propionic, n-butyric, and isobutyric acids.

6. The process of claim 5 wherein the carboxylic acid is acetic acid having a purity within the range of 80 to 95%.

7. The process of claim 5 wherein the carboxylic acid is acetic acid, the dicyclopentadiene is DCPD, and the tricyclodecenyl ester is tricyclodecenyl acetate (TCDA).

8. The process of claim 7 wherein the DCPD is added gradually to a mixture comprising acetic acid and triflic acid.

9. The process of claim 7 wherein a TCDA-rich product is recovered by distillation.

10. The process of claim 9 wherein the product comprises at least 95 wt.% of TCDA isomers.

11. The process of claim 10 wherein the product has an index of refraction (n_{D}²⁰) within the range of 1.49 to 1.50.

12. The process of claim 10 wherein the product has a specific gravity (d₄²⁰) within the range of 1.07 to 1.08.

13. The process of claim 1 wherein the amount of triflic acid is less than 1 wt.% based on the amount of the dicyclopentadiene.

14. The process of claim 1 wherein the reaction is performed at a temperature within the range of 60°C to 150°C.

15. The process of claim 1 performed in batch, semi-continuous, or continuous mode.

## Patentansprüche

1. Verfahren umfassend ein Reagieren einer C₂ - C₄ Carbonsäure und eines Dicylopentadiens bei einem Molverhältnis von Carbonsäure: Dicyclopentadien in einem Bereich von 0,8 bis 1,3 in Anwesenheit einer Trifluormethansulfonsäure unter geeigneten Bedingungen zur Herstellung eines Tricyclodecenylesters.

2. Verfahren nach Anspruch 1, wobei das Dicyclopentadien ein unsubstituiertes Dicyclopentadien (DCPD) ist.

3. Verfahren nach Anspruch 1, wobei das Dicyclopentadien eine Reinheit im Bereich von 80 bis 90 Prozent aufweist.

4. Verfahren nach Anspruch 1, wobei der Tricyclodecenylester destilliert wird zur Herstellung einer kommerziell akzeptablen Parfümkomponente.

5. Verfahren nach Anspruch 1, wobei die Carbonsäure ausgesucht ist aus einer Gruppe bestehend aus Essigsäure, Proprionsäure, n-Buttersäure und Isobuttersäure.

6. Verfahren nach Anspruch 5,wobei die Carbonsäure eine Essigsäure mit einer Reinheit im Bereich von 80 bis 95 Prozent ist.

7. Verfahren nach Anspruch 5, wobei die Carbonsäure eine Essigsäure, das Dicyclopentadien DCPD und der Tricyclodecenylester Tricyclodecenylacetat (TCDA) ist.

8. Verfahren nach Anspruch 7, wobei das DCPD schrittweise zu einer Mischung aus Essigsäure und Trifluormethansulfonsäure zugegeben wird.

9. Verfahren nach Anspruch 7, wobei ein TCDA-reiches Produkt durch Destillation zurückgewonnen wird.

10. Verfahren nach Anspruch 9, wobei das Produkt zumindest 95 Gewichtsprozent des TCDA-Isomers enthält.

11. Verfahren nach Anspruch 10, wobei das Produkt einen Brechungsindex (n_{D}²⁰) im Bereich von 1,49 bis 1,50 aufweist.

12. Verfahren nach Anspruch 10, wobei das Produkt eine relative Dichte D₄²⁰ im Bereich von 1,07 bis 1,08 aufweist.

13. Verfahren nach Anspruch 1, wobei die Menge an Trifluormethansulfonsäure weniger als 1 Gewichtsprozent basierend auf der Menge von Dicyclopentadien ist.

14. Verfahren nach Anspruch 1, wobei die Reaktion bei einer Temperatur im Bereich von 60 bis 150 °C durchgeführt wird.

15. Verfahren nach Anspruch 1, durchgeführt im diskontinuierlichen, halbkontinuierlichen oder kontinuierlichen Modus.

## Revendications

1. Procédé comprenant la réaction d'un acide C₂-C₄ carboxylique et d'un dicyclopentadiène à un rapport molaire acide carboxylique : dicyclopentadiène dans la plage de 0,8 à 1,3 en présence d'acide triflique dans des conditions efficaces pour produire un ester de tricyclodécényle.

2. Procédé selon la revendication 1 où le dicyclopentadiène est le dicyclopentadiène non substitué (DCPD).

3. Procédé selon la revendication 1 où le dicyclopentadiène a une pureté dans la plage de 80 à 90 %.

4. Procédé selon la revendication 1 où l'ester de tricyclodécényle est distillé pour produire un composant de parfum commercialement acceptable.

5. Procédé selon la revendication 1 où l'acide carboxylique est choisi dans le groupe consistant en les acides acétique, propionique, n-butyrique et isobutyrique.

6. Procédé selon la revendication 5 où l'acide carboxylique est l'acide acétique ayant une pureté dans la plage de 80 à 95 %.

7. Procédé selon la revendication 5 où l'acide carboxylique est l'acide acétique, le dicyclopentadiène est le DCPD et l'ester de tricyclodécényle est l'acétate de tricyclodécényle (TCDA).

8. Procédé selon la revendication 7 où le DCPD est ajouté progressivement à un mélange comprenant de l'acide acétique et de l'acide triflique.

9. Procédé selon la revendication 7 où un produit riche en TCDA est recueilli par distillation.

10. Procédé selon la revendication 9 où le produit comprend au moins 95 % en poids d'isomères de TCDA.

11. Procédé selon la revendication 10 où le produit a un indice de réfraction (n_{D}²⁰) dans la plage de 1,49 à 1,50.

12. Procédé selon la revendication 10 où le produit a une densité (d₄²⁰) dans la plage de 1,07 à 1,08.

13. Procédé selon la revendication 1 où la quantité d'acide triflique est inférieure à 1 % en poids sur la base de la quantité de dicyclopentadiène.

14. Procédé selon la revendication 1 où la réaction est conduite à une température dans la plage de 60°C à 150°C.

15. Procédé selon la revendication 1 mis en oeuvre en mode discontinu, semicontinu ou continu.
